Europäisches Patentamt

European Patent Office

Office européen des brevets

(11) Publication number: **0 359 310**
**A1**

## (12) EUROPEAN PATENT APPLICATION

(21) Application number: **89202232.8**

(22) Date of filing: **05.09.89**

(51) Int. Cl.5: **A61K 35/78**

(30) Priority: **15.09.88 US 245098**

(43) Date of publication of application:
**21.03.90 Bulletin 90/12**

(84) Designated Contracting States:
**AT BE CH DE ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Cupps, Thomas Lee**
**405 Pamela Drive**
**Oxford, OH 45056(US)**

(74) Representative: **Suslic, Lydia et al**
**Procter & Gamble European Technical**
**Center N.V. Temselaan 100**
**B-1820 Strombeek-Bever(BE)**

(54) Extracts of mandevilla pentlandiana useful as bradykinin antagonists.

(57) The present invention involves extracts of Mandevilla spentlandiana having bradykinin antagonist activity.

Effect of M. pentlandiana Ethyl Acetate Extract
in the Rat Uterine Contraction Assay

## EXTRACTS OF MANDEVILLA PENTLANDIANA USEFUL AS BRADYKININ ANTAGONISTS

Technical Field

The present invention relates to novel extracts of Mandevilla pentlandiana which act as antagonists of the biological activities of bradykinin.

Background of the Invention

Bradykinin, and its physiologically important related peptides kallidin (lysine-bradykinin) and methionine-lysine-bradykinin, contract smooth muscle (for example to produce diarrhea and inflammatory bowel disease and asthma); dilate vascular smooth muscle; lower blood pressure; mediate inflammation, as in allergies, arthritis and asthma; participate in blood clotting and complement-mediated reactions in the body; mediate rhinitis (viral, allergic and non-allergic); and are overproduced in pathological conditions such as septic shock, acute pancreatitis, hereditary angioneurotic edema, post gastrectomy dumping syndrome, carcinoid syndrome, anaphylactic shock, reduced sperm motility and certain other conditions. The production of bradykinin results in pain at the site of the pathological condition and the overproduction of bradykinin intensifies the pain directly via stimulation of sensory nerve fibers, or by the activation of the arachidonic acid pathway, which produces prostaglandins and leukotrienes and/or by release of other neuropeptides. Bradykinin and bradykinin-related kinins are not only produced by the animal, but may also be injected as a result of stings and bites. It is known that insects inch as hornets and wasps inject bradykinin-related peptides which also cause pain, swelling and inflammation. Literature references describing these actions of bradykinin and related peptides are found in the Handbook of Experimental Pharmacology, Vol. 25 (1970) and Vol. 25 Supplement (1979), Springer-Verlag.

The existence, activity and peptide structure of bradykinin has been known since about 1960 (Boissonnas, R.A., S. Guttman, P. A. Jaquenoud, H. Konsett & E. Sturmer, "Synthesis and Biological Activity Peptides Related to Bradykinin", Experimenta, Vol. 16 (1960), 326). Bradykinin is a naturally occurring agonist which binds to one or are specific biological receptors and stimulates pain and inflammatory responses and smooth muscle contraction in mammals, including man. Since elucidation of the bradykinin structure as a nonapeptide, there has been speculation that peptides with a similar structure could act as bradykinin antagonists by binding to the bradykinin receptor without stimulating the pain and inflammatory responses of tissues. Several hundred such sequence-related peptide analogs have been synthesized and assayed in biological systems: Stewart, J. M., & D. W. Woolley, "The Search for Peptides with Specific Anti-Bradykinin Activity", Hypotensive Peptides, Springer-Verlag, New York, Inc. (1966), pp. 23-33; Schroeder, E., "Structure-Activity Relationships of Kinins", Handbook of Experimental Pharmacology, Vol. 25 (1970), Springer-Verlag, pp. 324-350; Stewart, J. M., "Chemistry and Biologic Activity of Peptides Related to Bradykinin", Handbook of Experimental Pharmacology, Vol. 25 Supplement (1979), Springer-Verlag, pp. 227-272; Stewart, J. M., & R. J. Vavrek, "Bradykinin Chemistry: Agonists and Antagonists", Advances in Experimental Medicine and Biology, Vol. 156 (1983), pp. 585-589; Stewart, J. M., & R. J. Vavrek, U. 5. Patent No. 4,693,993, September 15, 1987.

Extracts from Mandevilla velutina have been shown to have activity as bradykinin antagonists: Calixto, J. B., M. Nicolau & R. A. Yunes, "A Selective Antagonist of Bradykinin Action from a Crude Extract of Mandevilla velutina. Part II. Effect on Nonuterine Smooth Muscles", Brazilian Journal of Medical and Biological Research, Vol. 18 (1985), p. 729A; Calixto, J. B., M. Nicolau & R. A. Yunes, "The Selective Antagonism of Bradykinin Action on Rat Isolated Uterus by Crude Mandevilla velutina Extract", British Journal of Pharmacology, Vol. 85 (1985), pp. 729-731; Calixto, J. B., & R. A. Yunes, "Effect of a Crude Extract of Mandevilla velutina on Contractions Induced by Bradykinin and [des-Arg$^9$]-Bradykinin in Isolated Vessels of the Rabbit", British Journal of Pharmacology, Vol. 88 (1986), pp. 937-941; Calixto, J. B., M. Nicolau, M. G. Pizzolatti & R. A. Yunes, "Kinin Antagonist Activity of Compounds from Mandevilla velutina in the Rat Isolated Uterus", British journal of Pharmacology, Vol. 91 (1987), pp. 199-204; and Calixto, J.B., M. B. Pizzolatti & R. Yunes, "The Competitive Antagonistic Effect of Compounds from Mandevilla velutina on Kinin-induced Contraction of Rat Uterus and Guinea Pig Illeum in vitro", British Journal of Pharmacology, Vol. 94 (1988), pp. 1133-1142. These researchers determined that at least five different compounds in the extracts of M. velutina possess bradykinin antagonist activity, and that these compounds are possibly terpene glycosides.

It is an object of the present invention to provide novel compositions having activity as bradykinin antagonists.

It is another object of the present invention to provide novel methods for providing analgesia and/or smooth muscle relaxation, and for treating inflammation, rhinitis, rhinorrhea, sore throat pain, congestion and/or other conditions mediated by bradykinin, by administering such bradykinin antagonist compositions to humans or lower animals.

## Summary of the Invention

The present invention relates to bradykinin antagonist compositions which are crude or purified extracts of Mandevilla pentlandiana.

## Detailed Description of the Invention

In addition to Mandevilla velutina (M. pohliana) extracts, which have been reported to have bradykinin antagonist activity by Calixto, et al., samples of four other species of Mandevilla were obtained and extracts of them were made and tested for bradykinin antagonist activity. The extracts of only one of these additional species, Mandevilla pentlandiana exhibited substantial bradykinin antagonist activity. Extracts of M. pentlandiana having bradykinin antagonist activity are primarily obtained from the underground plant structures, including stems, rhizomes, roots and associated root structures such as tubers.

Extracts of M. pentlandiana having bradykinin antagonist activity are preferably obtained by comminuting the underground portions of the plant in an organic or aqueous/organic solvent and separating the resulting liquid from the solid plant materials. Non-limiting examples of solvent systems that may be used to obtain extracts of M. pentlandiana include ethanol/water mixtures, chloroform, methylene chloride, and ethyl acetate. Preferred solvent systems include 80% ethanol/20% water and ethyl acetate. The most preferred solvent for obtaining extracts of M. pentlandiana is ethyl acetate.

The extracts of M. pentlandiana are useful as is as bradykinin antagonists or may be purified to obtain materials of greater activity and/or having fewer other substances in the extract which may cause unwanted side effects. Well known procedures for purifying materials can be used for purifying the extracts of M. pentlandiana. Such procedures include, without limitation, extraction, distillation, crystallization, various chromatography processes, and combinations thereof. The test methods disclosed hereinbelow can be used to select the fractions of purified extracts having greater bradykinin antagonist activity.

Compositions of the present invention include crude or purified extracts of M. pentlandiana themselves, and mixtures comprising such extracts and a pharmaceutically-acceptable carrier. Preferably such compositions are in dosage unit form. Such compositions typically comprise from about 0.0005% to 20% by weight of a crude or purified extract of M. pentlandiana, preferably from about 0.01% to about 10% of crude extract, more preferably from about 0.1% to about 1% of crude extract, or preferably from about 0.001% to about 5% of purified extract, more preferably from about 0.005% to about 0.5% of purified extract.

The term "pharmaceutically-acceptable carrier" as used herein, means one or more compatible solid or liquid filler diluents or encapsulating substances which are suitable for administration to a human or lower animal. The term "compatible" as used herein, means that the components of the pharmaceutical carrier are capable of being commingled with the extracts of M. pentlandiana, in a manner such that there is no interaction which would substantially reduce the pharmaceutical efficacy of the composition under ordinary use situations. Pharmaceutically-acceptable carriers must, of course, be of sufficiently high purity and sufficiently low toxicity to render them suitable for administration to the human or lower animal being treated.

Some examples of substances which can serve as pharmaceutically-acceptable carriers are sugars such as lactose, glucose and sucrose; starches such as corn starch and potato starch; cellulose and its derivatives, such as sodium carboxymethyl-cellulose, ethylcellulose, cellulose acetate; powdered tragacanth; malt; gelatin; talc; stearic acid; magnesium stearate; calcium sulfate; vegetable oils such as peanut oil, cottonseed oil, sesame oil, olive oil, corn oil and oil of theobroma; polyols such as propylene glycol, glycerine, sorbitol, mannitol, and polyethylene glycol; alginic acid; pyrogen-free water; isotonic saline; phosphate buffer solutions; cocoa butter (suppository base); emulsifiers, such as the TWEENS®; as well as other non-toxic compatible substances used in the pharmaceutical formulations. Wetting agents and lubricants such as sodium lauryl sulfate, as well as coloring agents, flavoring agents, excipients, tableting agents, stabilizers, antioxidants, and preservatives, can also be present.

Preferred compositions of the present invention include compositions for oral ingestion, e.g., tablets, capsules, powders, liquids (including sprays), suspensions, emulsions, lozenges, pastes, and the like; compositions for inhalation, e.g., mists, sprays, vapors, and the like; compositions for topical application, e.g., lotions, creams, ointments, salves, powders, gels, sticks, and the like; compositions for parenteral injection, e.g., liquids, suspensions, emulsions, and the like. More preferred compositions are compositions for oral ingestion, inhalation, or topical application; most preferred are compositions for oral ingestion.

Methods for Determining Bradykinin Antagonist Activity

The following test methods describe procedures used to measure the bradykinin antagonist activity of the extracts, purified extracts and compositions of the present invention. Preferred materials and methods for obtaining the extracts and purifying them can be determined by measuring the bradykinin antagonist activity of the various extracts, purified extracts and compositions using the following methods.

Test Method 1

Guinea Pig Ileum Assay

The bradykinin antagonists are assayed on guinea pig ileum for inhibition of the muscle-contracting activity of bradykinin, according to the commonly accepted assay method for bradykinin and related kinins as described in Stewart, J.M., "Chemistry and Biological Activity of Peptides Related to Bradykinin", Chapter V of Bradykinin Kallidin and Kallikrein, (Handbook of Expt. Pharmacol.), E. G. Erdoes (ed.), Vol 25, Springer Verlag (pub.), 1970, pp. 242-243. The inhibition potencies are determined on isolated guinea pig ileum, according to the commonly accepted manner described for antagonists of biologically active compounds in Schild, H.O., "pA2, a New Scale for the Measurement of Drug Antagonism", British Journal of Pharmacology, Vol. 2, No. 3 (1947), pp. 189-206). In the assay, a dose-response curve is determined for the reference substance bradykinin. The dose of bradykinin which produces a half maximal contraction of tissue is the $ED_{50}$ dose. An amount of bradykinin equivalent to twice the $ED_{50}$ dose is administered to the tissue 30 seconds after the start of incubation of the tissue with a dose of antagonist. Doses of antagonist are increased in this protocol until pre-incubation with a dose of antagonist reduces the contraction in response to the double $ED_{50}$ dose of bradykinin to response of a single $ED_{50}$ dose of bradykinin. The pA2 value represents the negative logarithm of the molar concentration of antagonist which, in its presence, requires twice the amount agonist to elicit the same response as when the agonist is given by itself. One unit of pA2 value represents an order of magnitude change in potency. For comparison, the negative log of the dose of bradykinin, the dose which causes half maximal contraction of the tissues, is commonly known as the pD2 value. The pD2 value for bradykinin is 7.4 on the guinea pig ileum.

Test Method 2

Plasma Extravasation in the Guinea Pig Trachea

Male Hartley guinea pigs weighing 450-700 g are anesthetized with sodium pentobarbital. The right

4

jugular vein is catheterized with a silastic tubing filled with 0.9% saline. The right carotid artery is cannulated. Evans blue dye is administered slowly via the jugular vein catheter. Five minutes later, bradykinin (0.4 ml/kg) and a test analog (0.4 ml/kg) are co-administered intraarterially. Following an additional five minute period, the vasculature is perfused via the jugular vein with 0.9% saline. A 1 cm section of tracheal tissue directly above the bifurcation is removed and weighed. Tissue is placed in tubes containing formamide. The tubes are placed in 50°C waterbath for 24 hours. Samples of the solvent from the tubes are transferred to cuvettes and measurements are taken on a fluorescence spectrophotometer. Calculations are made to determine dye content in the tissue. Data is expressed as ng dye/mg tissue.

Test Method 3

Mouse Abdominal Constriction Test

Compounds of the present invention are tested for analgesic activity using the mouse abdominal constriction assay. This assay is described in Hendershot, L.C., & J. Forsaith, "Antagonism of the Frequency of Phenylquinone-induced Writhing in the Mouse by Weak Analgesics and Nonanalgesics", Journal of Pharmacology, Vol. 125, (1959), pp. 237-240, and in Methods in Narcotics Research, S. Ehrenpreis and A. Neidle, (eds.), Marcel Dekker, Inc., New York (pub.), 1975, pp. 64-65, the disclosures of both these references being incorporated herein in their entirety by reference.

Male CF-1 mice (Charles River Breeding Laboratories, Inc.), weighing approximately 20 grams are used in these assays. Test compounds are prepared for intraperitoneal (i.p.) injection, with compounds of the present invention being prepared in ethanol:Tween 80:acidified deionized distilled water (10:10:80, v/v/v) so that the appropriate dose is given in 0.2 mls to a 20 gram mouse.

An i.p. injection of the test compound is given. Ten minutes later, an i.p. injection of 0.02% phenylquinone (2.5 mg/kg) is given at a dosing volume of 0.25 ml/20 grams body weight. Ten minutes after the i.p. injection of the phenylquinone, the number of full body writhes is counted for the succeeding ten minutes. Percent analgesia in this assay is calculated as follows:

$$\frac{\text{Control number of writhes - Dosed number of writhes}}{\text{Control number of writhes}} \times 100$$

Test Method 4

Rat Uterine Contraction Assay

The procedures described below are primarily from two sources: Kitchen, I., Textbook of In Vitro Practical Pharmacology, Blackwell Scientific Publications, Oxford, 1984; and Pharmacological Experiments on Isolated Preparations, The Department of Pharmacology, University of Edinburgh, Churchill Livingstone, 1977.

The methods employed in the use of isolated tissue preparations are described below.

Tissue Baths

10 ml detachable jacketed glass organ baths with joint and springs (Harvard Apparatus Bioscience) are used in this preparation. (Note: based on measurements of actual internal volume, all calculations are based on a 15 ml tissue bath volume.) The bath consists of an internal chamber with an inflow and an outflow for the buffer solution and a scinter glass at its base to allow aeration. This is jacketed by an outer warming jacket connected to a circulating water bath. There is also a port which allows for overflow. The buffer is circulated through a glass coil contained in a 30°C water bath prior to reaching the tissue baths.

The tissue is secured within the bath by two stainless steel curved needles. One is attached at the base, and the other is removable and has a loop for connection to the transducer.

The tissue is washed prior to and following dosing of the positive control drug (usually acetylcholine), and after cumulative dose-responses are complete. Washing consists of draining the buffer solution from the bath via the drain outlet port, and allowing inflow of fresh, warmed buffer solution via the buffer inflow port. Typically each "wash" is performed three times in this manner. ,

Physiological salt solution

De Jalon buffer solution, prepared in distilled water, is used in this preparation:

| Component | Final Concentration (mM) |
|---|---|
| Sodium chloride (NaCl) | 154.0 |
| Potassium chloride (KCl) | 5.6 |
| Calcium chloride ($CaCl_2 \cdot 2H_2O$) | 0.3 |
| Magnesium chloride ($MgCl_2 \cdot 6H_2O$) | 1.4 |
| Sodium bicarbonate ($NaHCO_3$) | 6.0 |
| Dextrose | 2.8 |

1 M stock solutions ($CaCl_2$ and $MgCl_2$) are stored refrigerated (4°C) for up to two weeks. Concentrated buffer stock (NaCl 90 g/l, KCl 4.2 g/l, and $NaHCO_3$ 5 g/l) is stored frozen (0°C) for up to two weeks. The final buffer preparation is performed on the day of the experiment and consists of the following: One part concentrated buffer stock solution is added to 9 parts distilled water. Dextrose (0.5 g/l), 1M $CaCl_2$ (0.3 ml/l), and 1M $MgCl_2$ (1.2 ml/l) are then added to the diluted buffer. The buffer is aerated with 95% $O_2$:5% $CO_2$ for 1 hour.

Recording transducer

Contraction of the rat uterus smooth muscle is measured isometrically on an 8-channel Grass Model 7 Recorder using Grass FT.03 force transducers, Grass model 7P1 Low-Level D.C. Preamplifiers, and Grass model 7DA Driver Amplifiers. Strain on the transducer is converted into an electrical potential which is amplified and recorded as a pen movement on a chart recorder tracer.

Resting tension, equilibration, and instrument standardization

The tissue is placed under a resting tension of 0.5 g. The recorder baseline is set 0.5 cm above the maximum lower point of pen movement. Tissues are allowed to equilibrate for approximately 60 minutes.

Standardization, performed prior to each experiment, adjusts the electronic balance between the driver amplifier and the pre-amplifier for each channel and sets the pen positions for a known baseline tension deflection. With the driver amplifier on, and the polarity switch on Up Cal, the baseline is set using the baseline position control knob. The driver cal red button is pressed, and the pen should deflect 2 cm from baseline. This deflection may be adjusted with the driver sensitivity control knob. Three successful readings complete the standardization of the driver.

Resting load weights of 0.5g are placed on the force transducers and the polarity switch turned to Use. The sensitivity is typically set on 0.2 or 0.5 mv/cm. The balance voltage adjustants (coarse and fine) will adjust the pens to baseline. A 4 g weight is then placed on each transducer (in addition to the 0.5 g resting load weight). The pens are then adjusted using the pre-amplifier sensitivity control knob to a 4 cm deflection (e.g., 4 g = 4 cm at 0.2 mv/cm).

Control and test sample preparations

Acetylcholine (ACh) is obtained from Sigma Chemical Company, No. A-6625, FW 181.7.

| Final Concentration | Dose Volume | Initial Concentration | Vehicle |
|---|---|---|---|
| 100 μM | 75 μl | 20 mM (3.64 mg/ml) | De Jalon buffer |

ACh dosing solution is prepared fresh daily, kept at 4°C and protected from light.

Bradykinin (Bk) is obtained from BaChem Inc., 10 mg vials, FW 1060. A 2 mM stock solution is prepared by dissolving 10 mg Bk in 4.71 ml of 0.01 M acetic acid. This solution is then divided into 300 μl aliquots which are placed in Eppendorf centrifuge tubes and frozen (0°C).

On the morning of each experiment, a series of Bk solutions of the following concentrations (μM) are prepared: 0.0001, 0.0003, 0.0001, 0.0003, 0.01, 0.03, 0.1, 0.3, 1, 3, 10, 30. The vehicle for each Bk dosing solution is De Jalon buffer.

Test samples are prepared by either suspending the desired quantity of crude or purified extract in de Jalon buffer or dissolving it in dimethylsulfoxide (DMSO).

Measuring responses

These experiments are based on the ability of isolated muscle strips to produce dose-related contractions in response to added drugs. The force of contraction is measured as the distance from the steady baseline to the top of the first peak in the drug-induced response. This distance is converted to g of force using the known relationship between pen deflection and force. It is later converted to a percentage of the response to 100 μM ACh. In the cumulative dosing method, each incremental dose is added to the tissue bath just after the maximal effect of the preceding dose is obtained.

Animals

Sprague-Dawley female rats are obtained from Charles River Breeding Laboratories, Inc., Portage, Michigan. Animals weigh between 125-150 g.

Twenty-four hours prior to use, a sexually mature female rat is injected subcutaneously with 0.1 mg/kg (1 ml/kg) diethylstilbestrol (DES) in ethanol or sesame oil. DES is obtained from Sigma Chemical Company, D-4628, FW 268.4. A 25 mg/ml ethanolic stock solution is prepared and stored frozen (0°C) and protected from light for up to two weeks.

Tissue preparation

A rat is anesthetized with sodium pentobarbital unless otherwise noted. The abdomen is opened and the uterine horns exposed by pulling aside the intestines. The ovaries are located and lifted away with the uterine horns from surrounding fat and mesenteric attachments. The excised tissue is placed in a petri dish containing De Jalon buffer warmed to 30°C and continuously oxygenated with 95% $O_2$:5% $CO_2$. The tissue is cut transversely into four segments. A curved needle is attached to either end of the segment. The segment is then suspended in a longitudinal orientation in the tissue bath by connecting the suture loop at one end of the tissue to a hook fixed to the base of the tissue bath. The opposite suture is hooked to the transducer. Following mounting, the resting tension of the muscle strip is adjusted to 0.5 g by adjusting the force transducer assembly.

Experimental procedure

Following equilibration, the tissues are treated with acetylcholine ($10^{-4}$M) and DMSO (amount equal to that which will be added with test sample, if any). This test ensures tissue viability and establishes an

7

internal standard response against which all further responses can be measured. Three successive washes are performed and the tissues allowed to relax back to baseline. A single dose of Bk (1 μM) is given at this time; the tissue is washed and allowed to relax again. A second and third ACh response is obtained prior to the Bk cumulative dose-response. Baseline tension is readjusted to 0.5 g between each test as necessary.

An initial cumulative dose-response curve is obtained with several Bk doses ($10^{-10}$ to $10^{-5}$ M). Three washes are performed on each bath and the tissues are allowed to relax. The tissues are then contracted with 100 μM ACh and washed three more times. Thirty minutes following the initial Bk dose, the tissues are pre-treated with a test sample for 20 minutes. Another cumulative Bk dose-response curve is obtained in the presence of the test sample. Three washes are performed, ACh and DMSO dosed, washed out and the tissue allowed to relax for approximately 30 minutes. A final cumulative Bk dose-response curve is obtained. The tissues are washed, and a final ACh response is obtained.

Data analysis

The contractile forces at each dose are entered into a Lotus 1-2-3 spreadsheet. The data from each dose-response determination are normalized to the 100 μM ACh response using the formula:

$$\% \text{ of Maximal Response} = \frac{\text{Response (Bk)}}{\text{Response (ACh)}} \times 100\%$$

where Response (ACh) is the average of the ACh responses immediately prior to and following the determination of the Bk response. The mean and standard deviation of the raw force data and the normalized data at each dose are calculated, and the mean values are stored in a file for further analysis. The successive dose-response curves are then analyzed for differences in shape (height, slope) or location ($ED_{50}$ value) using a 4-parameter logistic curve analysis program, ALLFIT, as described in DeLean, A., P. J. Munson & D. Rodbard, "Simultaneous Analysis of Families of Sigmoidal Curves: Application to Bioassay, Radioligand Assay, and Physiological Dose-Response Curves", American Journal of Physiology, Vol. 235 (1978), pp. E97-E102. The following equation is used to model the data:

$$Y = \frac{a-d}{1 + (x/c)^b} + d$$

where Y is the response; x, the arithmatic dose; a, the response when x = 0; d, the response for "infinite" dose, c is the $ED_{50}$, i.e., the dose resulting in a response halfway between a and d; and b is a "slope factor" that determines the steepness of the curve. The factor a is typically forced to zero to produce curves which extrapolate to zero at low doses of Bk. The data and appropriately fitted curves are then plotted using GRAFIT, a companion plotting program for ALLFIT.

Example 1

An extract of M. pentlandiana root material and associated beneath-ground structures is prepared by extracting 1800 g of the dried, crushed root with 4000 g of ethyl acetate and evaporating the product to dryness in a rotary evaporator.

According to test method 4 above, a 468 mg portion of this material is suspended in 5 ml of de Jalon buffer using a high shear mixer (Pc'ytron®) to achieve a uniform dispersion. One-half milliliter aliquots of this suspension are dosed to each of four 15 ml tissue baths according to the test method 4 procedure described above, producing a concentration of 3.1 mg of dried extract/ml of bath solution.

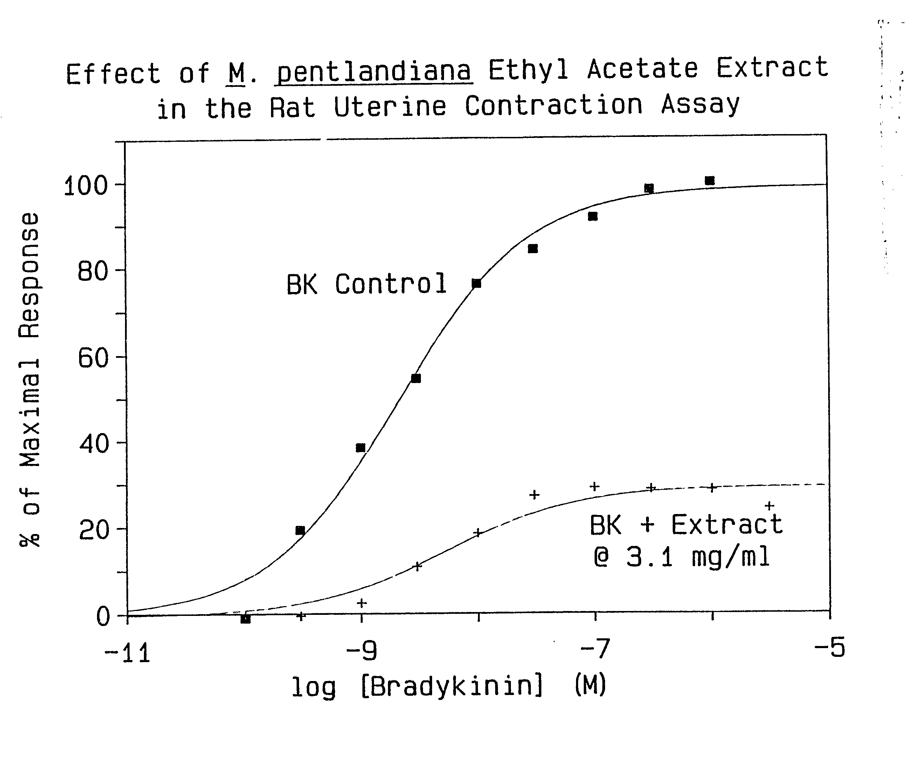

Bradykinin (Bk) dose-response curves for the rat uterine strips before and after addition of the root extract are obtained. The results are shown in the Figure. The $EC_{50}$ ratio for Bk in the presence of extract versus the Bk control is 2.6 ± 1.2 (mean ± SE), and the ratio of the dose-response curve maxima is 0.30 ±

Example 2

According to test method 3, M. pentlandiana extract from Example 1 is dissolved in ethanol:Tween 80:acidified deionized distilled water (10:10:80 v/v/v) at a concentration of 3 mg/ml. Male CF-1 mice, weighing 15-20 g, are given an intraperitoneal (i.p.) injection of 0.2 ml/20 g (body weight) (a dose of 30 mg/kg) 10 minutes prior to i.p. injection of 0.02% p-phenylquinone (.25 ml/20 g). The mice are then placed in individual shoe box cages. Abdominal constrictions, full body stretches with hindlimbs extended behind the body, are counted for 10 minutes after the phenylquinone injection (20 minutes after M. pentlandiana extract).

The results are as follows:

| Treatment | Avg # Constrictions | (n) |
|---|---|---|
| Vehicle | 10.4 ± 2 | (8) |
| M. pentlandiana | 1.3 ± 0.9 | (8) |

M. pentlandiana extract produces an 88% decrease in the number of abdominal constrictions produced by p-phenylquinone.

Another aspect of the present invention includes methods of producing anti-inflammatory activity and/or analgesia, smooth muscle relaxation, or treating rhinitis, rhinorrhea, and/or congestion in humans or lower animals by administering, to a human or lower animal in need of such treatment a safe and effective amount of a composition of the present invention. This safe and effective amount can be given in a single dose or multiple doses repeatedly over the course of the treatment. Potential methods of administering the compositions of the present invention include per oral, parenteral (e.g., intravenous, intramuscular, intraperitoneal, subcutaneous), topical (epithelial, gingival, dermal or transdermal), intranasal, inhalation, sublingual, intravaginal, intrarectal, intrabuccal, or other internal administration means. Preferred methods of administering the compositions of this invention include per oral, parenteral, inhalation and topical; more preferred are per oral, inhalation and topical; most preferred is per oral.

The phrase "safe and effective amount", as used herein, means an amount of a composition of the present invention high enough to significantly modify the condition to be treated in a positive way, but low enough to avoid serious side effects (at a reasonable benefit/risk ratio), within the scope of sound medical judgement. The safe and effective amount of the composition will vary with the particular condition being treated, the age and physical condition of the patient being treated, the severity of the condition, the duration of the treatment, the nature of concurrent therapy, the specific composition employed, the particular pharmaceutically-acceptable carrier utilized, and like factors within the knowledge and expertise of the attending physician.

The compositions of this invention are useful for the treatment of one or more of the traumatic, inflammatory, pathological or normal conditions which are known to be mediated by Bk or exacerbated by an overproduction of Bk. These conditions involve pain, inflammation, congestion and/or smooth muscle contractions. Such conditions include wounds, burns, rashes, snake bites, insect bites and stings, angina, arthritis, asthma, allergies, rhinitis, rhinorrhea, sore throat pain, congestion, shock, inflammatory bowel disease, low blood pressure, systemic treatment of pain and inflammation, and/or low sperm motility which produces male infertility.

Therapeutic applications of the Bk antagonist extracts and compositions of the present invention include not only treatment for the production of Bk or related kinins by the animal but also the injection of Bk related peptides into an animal as a result of bites and stings.

While particular embodiments of the present invention have been described, it will be obvious to those skilled in the art that various changes and modifications to the compositions disclosed herein can be made without departing from the spirit and scope of the invention. It is intended to cover, in the appended claims, all such modifications that are within the scope of this invention.

## Claims

1. An extract of tissues of the plant species Mandevilla pentlandiana characterized in that it has bradykinin antagonist activity.

2. The extract of Claim 1 characterized in that the tissues from which the extract is made comprise underground plant structures.

3. A purified extract derived from the extract of Claim 1 or 2 characterized in that the purified extract has greater bradykinin antagonist activity than the extract.

4. A pharmaceutical composition characterized in that it comprises an extract of any of Claims 1-3 and a pharmaceutical carrier.

5. The composition of Claim 4 characterized in that it is in a form for oral ingestion.

6. The use of a safe and effective amount of the extract of any of Claims 1-3 for the manufacture of a medicament to treat pain, inflammation, smooth muscle contraction or congestion in a human or lower animal in need of such treatment.

**Effect of <u>M</u>. <u>pentlandiana</u> Ethyl Acetate Extract in the Rat Uterine Contraction Assay**

BK Control

BK + Extract @ 3.1 mg/ml

% of Maximal Response

log [Bradykinin] (M)

EP 0 359 310 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 99, no. 17, 24th October 1983, page 345, abstract no. 136862j, Columbus, Ohio, US; E.L. MICHELOTTI et al.: "Phytochemical study of Mandevilla pentlandiana. I. Hydrocarbons, lipids, sterols, and triterpenols in the petroleum ether extract" & AN. ASOC. QUIM. ARGENT. 1983, 71(1), 179-83 | | A 61 K 35/78 |

TECHNICAL FIELDS SEARCHED (Int. Cl.5)

A 61 K

The present search report has been drawn up for all claims

| Place of search | Date of completion of the search | Examiner |
|---|---|---|
| THE HAGUE | 27-11-1989 | REMPP G.L.E. |